(19) Europäisches Patentamt
European Patent Office
Office européen des brevets

(11) EP 2 243 462 B1

(12) EUROPÄISCHE PATENTSCHRIFT

(45) Veröffentlichungstag und Bekanntmachung des Hinweises auf die Patenterteilung:
20.02.2013 Patentblatt 2013/08

(51) Int Cl.:
*A61K 8/06* (2006.01)      *A61K 8/34* (2006.01)
*A61K 8/37* (2006.01)      *A61Q 19/00* (2006.01)
*A61Q 19/10* (2006.01)

(21) Anmeldenummer: 09005530.2

(22) Anmeldetag: 20.04.2009

(54) **Emulgatorsystem**

Emulgator system

Système d'émulsifiant

(84) Benannte Vertragsstaaten:
AT BE BG CH CY CZ DE DK EE ES FI FR GB GR HR HU IE IS IT LI LT LU LV MC MK MT NL NO PL PT RO SE SI SK TR

(43) Veröffentlichungstag der Anmeldung:
27.10.2010 Patentblatt 2010/43

(73) Patentinhaber: Dr. Straetmans GmbH
22143 Hamburg (DE)

(72) Erfinder:
• **Jäninchen, Jan**
22149 Hamburg (DE)

• **Petersen, Wilfried**
22143 Hamburg (DE)
• **Salmina-Petersen, Manuela**
22143 Hamburg (DE)

(74) Vertreter: **Keussen, Christof**
**Glawe, Delfs, Moll & Partner**
**Patentanwälte**
**Rothenbaumchaussee 58**
**20148 Hamburg (DE)**

(56) Entgegenhaltungen:
EP-A- 1 426 029      EP-A- 1 911 436
EP-A- 1 946 742      EP-A- 2 005 937
WO-A-2007/013634    DE-A1-102005 051 864

**Beschreibung**

Die Erfindung betrifft ein Emulgatorsystem sowie dessem Verwendung bei der Herstellung kosmetischer oder dermatologischer Produkte.

Zur Herstellung kosmetischer oder dermatologischer Hautpflegeprodukte sind Emulgatoren von essentieller Bedeutung. Emulgatoren sind amphiphile Moleküle, welche sich aufgrund ihres molekularen Aufbaus in einem Zweiphasensystem bestehend aus einer polaren, wässrigen und einer unpolaren Ölphase an der Grenzschicht zwischen beiden Phasen anreichern. Sie reduzieren dadurch die Grenzflächenspannung zwischen den Phasen und ermöglichen je nach Charakter des Emulgators die Bildung von Öltröpfchen in wässriger Umgebung (O/W-Emulsion) oder Wassertröpfchen in einer Ölmatrix (W/O-Emulsion).

Der Charakter eines Emulgators kann nach Griffin über dessen Hydrophilic-Lipophilic-Balance-Wert, kurz HLB-Wert beschrieben werden (Griffin, W. C.: Classification of surface active agents by HLB, J. Soc. Cosmet. Chem. 1, 1949). Dieser Wert bietet eine mathematische Formel, auf deren Basis das Phasenverhalten von amphiphilen Molekülen vorhergesagt werden kann und berechnet sich gemäß:

$$\text{HLB} = 20 \times (1 - \text{Molekulargewicht des hydrophoben Molekül-teils} / \text{Gesamtmolekulargewicht})$$

Eine sehr wirkungsvolle Gruppe von Emulgatoren bilden Anlagerungsprodukte von Ethylenoxid oder Propylenoxid an lipophile Alkohole oder Säuren wie z.B. Fettalkohole (Laureth, Ceteareth), partiell hydrolysierte Glyceride (PEG-100 Glyceryl Stearat) oder Zuckerester (Tween®) (PEG-Emulgatoren) . Die photochemische Instabilität sowie die penetrationsfördernden Eigenschaften, welche diesen Emulgatortypen nachgesagt werden, haben neben deren petrochemischen Ursprung jedoch dazu geführt, dass in den vergangenen Jahren PEG-freie Alternativen entwickelt wurden.

Insbesondere solche Systeme, welche aus nachwachsenden Rohstoffquellen gewonnen werden können, erfreuen sich zunehmender Beliebtheit bei Verbrauchern und damit auch den Entwicklern kosmetischer und dermatologischer Produkte. Beispiele aus dieser Gruppe sind z.B. Zuckerester und Glucoside, Polyglycerinester, Acylderivate von Aminosäuren oder oligomerisierter Milchsäure (Lactylate), Phospholipide (Lecithine) oder Citrate von patiell hydrolysierten Glyceriden (z.B. Glyceryl Stearate Citrate).

Dem Fachmann ist bekannt, dass der Emulsionsbildungsprozess in der Regel nicht spontan erfolgt, sondern den Eintrag von thermischer Energie und Scherarbeit in das Zweiphasensystem erfordert. Da es sich bei den klassischen Emulsionen lediglich um kinetisch stabilisierte Systeme handelt, tragen die Emulgatoren darüber hinaus auch zur Stabilisierung derselben bei. In den meisten Fällen sind dabei neben den Emulgatoren jedoch noch weitere stabilisierende Hilfsstoffe notwendig, welche das Zusammenfließen der emulgierten Tröpfchen verhindern. So können die Ölphasen z.B. durch Zusatz von Fettalkoholen, welche flüssigkristalline Grundstrukturen aufbauen können, stabilisiert werden, während die wässrigen Phasen durch Gelbildner verdickt und dadurch die Koaleszenz der Tröpfchen verlangsamt wird. Auch Co-Emulgatoren aus der Gruppe der Monoglyceride langkettiger Fettsäuren sind zur Stabilisierung von Emulsionen geeignet.

Obwohl sich durch die gezielte Auswahl von Emulgatoren und Stabilisatoren das Erscheinungsbild und die haptischen Eigenschaften von kosmetischen Formulierungen fein justieren lassen, sind in den vergangenen Jahren zahlreiche fertige Abmischungen von Emulgatoren und Stabilisatoren auf den Markt gekommen, welche den Herstellprozess vereinfachen und die Anzahl der einzusetzenden Rohstoffe reduzieren sollen. So beschreibt EP-B2-0553241 selbstemulgierende Gemische aus Cetearyl Glucoside und Cetearyl Alkohol welche unter dem Namen Montanov® 68 (Seppic) bekannt sind. Die Fa. Gattefossé bietet unter dem Namen Emulium Kappa eine Mischung aus Candellila/ Jojoba/Rice Bran Polyglyceryl-3 Esters (and) Glyceryl Stearate (and) Cetearyl Alcohol (and) Sodium Stearoyl Lactylate an, welche besonders ansprechende Emulsionen ausbilden soll. Weitere marktübliche Systeme sind dem Fachmann bekannt.

Diese Mischungen beanspruchen, ohne weitere Co-Emulgatoren die Herstellung stabiler und ansprechender Emulsionen zu ermöglichen, deren Herstellung erfordert jedoch für gewöhnlich Temperaturen von 75 - 85°C.

Im Zuge weiterer Einsparungen von Prozesskosten und -zeiten verlangt der Markt jedoch auch nach Emulgatoren, welche bereits bei einem verringerten Energieeintrag die Bildung klassischer Emulsionen ermöglichen. Emulgatorsysteme, welche diese Anforderung erfüllen, sind zumeist flüssig.

So beschreibt EP-A1-1790327 ein flüssiges Emulgatorkonzept basierend auf einem oder mehreren Polyolpartialestern, einem oder mehreren neutralisierbaren Säurepartialestern und einem polaren Lösungsvermittler. Zur Stabilsierung dieser Emulsionen sind jedoch erfahrungsgemäß Gelbildner notwendig.

EP-B1-1641904 umfasst einen O/W-Emulgator bestehend aus Glyceryl Oleate Citrat und einem die Viskosität modifizierenden nativen Öl, die beschriebenen Beispielformulierungen werden jedoch vornehmlich bei Temperaturen von 70-85°C hergestellt und enthalten ebenfalls weitere Stabilisatoren.

Niedrigviskose O/W-Emulsionen können gemäß EP 1250961 A1 ebenfalls mit Mischungen von Polyglycerinestern und Sorbitanestern hergestellt werden. Die erhaltenen Emulsionen sind jedoch vielfach nicht lagerstabil oder müssen noch zusätzlich stabilisiert werden.

DE 10 2005 051 864 A1 offenbart eine kosmetische Öl-in-Wasser-Emulsion, die 1,2 Hexandiol enthält.

EP 2 005 937 A1 betrifft eine kosmetische O/W-Emulsion mit einem Gehalt an molekularem Sauerstoff, die durch eine bestimmte Ölzusammensetzung langsam und gleichmäßig Sauerstoff an die Haut abgeben soll.

WO 2007/013634 A1 offenbart, dass lagerstabile kosmetische O/W-Emulsionen erhalten werden, wenn ein Tocopherolglycinat-Derivat zusammen mit bestimmten Fettsäureestern verwendet wird. EP 1 911 436 A2 betrifft kaltherstellbare, langzeitstabile Emulsionen, die durch den Einsatz einer Polyglycerynester enthaltenden Emufgatormischung hergestellt werden.

Der Erfindung liegt die Aufgabe zu Grunde, ein festes Emulgatorsystem zur Verfügung zu stellen, das ohne weitere stabilisierende Zusätze und bei Temperaturen unterhalb der üblichen Temperaturen von 70-80°C die Herstellung stabiler Emulsionen mit einem ansprechenden Erscheinungsbild ermöglicht.

Die Erfindung löst diese Aufgabe durch die Merkmale der Patentansprüche. Ein erfindungsgemäßes festes Emulgatorsystem weist folgende Bestandteile auf:

a) mindestens einen Basisemulgator, ausgewählt aus der Gruppe der Ester oder Partialester von linearen oder verzweigten, gesättigten oder ungesättigten Fettsäuren mit einer Kettenlänge von n = 16 - 22 Kohlenstoffatomen, und einer Alkoholkomponente ausgewählt aus der Gruppe der Lactylate, der Polyglycerine mit einem Polymerisierungsgrad von n = 2 - 10 oder dem Zitronensäureesters des Glycerins,

b) mindestens einen Konsistenzgeber und/oder Co-Emulgator, ausgewählt aus der Gruppe der linearen oder verzweigten, gesättigten oder ungesättigten Fettalkohole mit einer Kettenlänge von n = 16 - 22 Kohlenstoffatomen und/oder den Monoglyceriden von linearen oder verzweigten, gesättigten oder ungesättigten Fettsäuren mit einer Kettenlänge von n = 16 - 22 Kohlenstoffatomen,

c) 2-15% mindestens eines Benetzmittels mit einem Molekulargewicht zwischen 100 und 250 g/mol und einem HLB-Wert zwischen 6 und 12, ausgewählt aus der Gruppe der Glycerinmonoester mit Fettsäuren einer Kettenlänge von n = 6 -10 Kohlenstoffatomen oder der 1,2-Alkandiole mit einer Kettenlänge n = 5 - 10 Kohlenstoffatomen.

Die erfindungsgemäßen Emulgatoren zeichnen sich durch die Bildung stabiler, ansprechender und feiner Emulsionen bei Temperaturen bereits deutlich unterhalb der herkömmlichen Emulsionsbildungstemperatur von 70-80°C aus.

Überraschenderweise wurde gefunden, dass die Einarbeitung schon geringer Konzentration eines Benetzmittels definierten Molekulargewichts und HLB-Wertes in ein festes, selbstemulgierendes Emulgatorsysteme, dessen Spontanemulgierbarkeit soweit verbessert, dass bereits bei Temperaturen von 40°C stabile Emulsionen sehr feiner Tröpfchenverteilung hergestellt werden können.

Die erfindungsgemäßen Emulgatorsysteme können durch gemeinsames Aufschmelzen der Bestandteile homogen vermengt und durch geeignete Prozesse beim Abkühlen in Form von Pellets, Schuppen, Granulaten oder Pulvern hergestellt werden. Stabile Emulsionen werden mit den erfindungsgemäßen Emulgatorsystemen erhalten, wenn diese bevorzugt in Konzentrationen von 2 - 10%, vorzugsweise 3 - 8%, weiter vorzugsweise 4 - 7%, weiter vorzugsweise 5 - 6% eingesetzt werden. Sämtliche Prozentangaben in der Beschreibung und den Patentansprüchen sind Gewichtsprozente, soweit nicht anders angegeben.

Besonders bevorzugt ist das erfindungsgemäße Emulgatorsystem PEG-frei, d.h. es enthält Polyethylenglykole nicht oder allenfalls als Verunreinigung.

Die durch die erfindungsgemäßen Emulgatorsysteme erhaltenen Emulsionen zeichnen sich durch ein sehr ansprechendes Hautgefühl und eine leicht Verteilbarkeit aus.

Darüber hinaus zeigte sich, dass die mikrobiologische Stabilität der von den erfindungsgemäßen Emulgatoren gebildeten Emulsionen gegenüber vergleichbaren Emulsionen, welche kein Benetzmittel enthalten, erheblich verbessert ist. Unter dem Begriff Benetzmittel werden amphiphile Moleküle zusammengefasst, die eine ausgeprägte Oberflächenaktivität aufweisen, deren Molekulargewicht jedoch deutlich unter denen klassischer Emulgatoren liegt. Daher eignen sich Benetzmittel zwar zur Erniedrigung von Grenzflächenspannung zwischen einer wässrigen und einer lipophilen Phase, nicht jedoch zur Herstellung stabiler Emulsionen. Vielmehr können diese Benetzmittel bei höheren Konzentrationen viskositätserniedrigend und emulsionszerstörend wirken. In niedrigen Konzentrationen eingebracht können Benetzmittel jedoch aufgrund ihrer Oberflächenaktivität die Ausbildung sehr homogener und feiner Emulsionen fördern. Als besonders effektiv haben sich in dieser Hinsicht amphiphile Moleküle mit einem Molekulargewicht von 100 - 250 g/mol und einem HLB nach Griffin von 6 - 12 herausgestellt. Auch besitzen diese Benetzmittel antimikrobielle Eigenschaften, welche Sie in einer kosmetischen Formulierung vorteilhaft ausbilden können.

Beispiele derartiger Benetzmittel findet man in der Gruppe der Monoglyceride von Fettsäuren mit einer Kettenlänge von 6 - 10 Kohlenstoffatomen oder der 1,2-Alkandiole mit einer Kettenlänge von 5 bis 10 Kohlenstoffatomen.

Marktübliche Emulgatorsysteme, welche keine weiteren Konsistenzgeber oder Co-Emulgatoren benötigen, werden für gewöhnlich durch Zusammenschmelzen der einzelnen Rohstoffkomponenten erhalten und als Pulver, Granulate, Schuppen oder Pellets angeboten. So kann man z.B. bei Temperaturen von 60-70°C Glyceryl Stearate Citrate mit Cetearylalkohol zusammenschmelzen und durch einen geeigneten Prozess in Form von festen Pellets isolieren. Auch kann man Polyglyceryl-3 Stearate mit Sodium Stearoyl Lactylate und Glyceryl Monostearate zusammenschmelzen. Eine Reihe weiterer marktüblicher Emulgatorsysteme wurde oben genannt.

Die erfindungsgemäßen Emulgatorsysteme eignen sich zur Herstellung von Emulsionen unterschiedlicher Viskosität wie Lotionen, Cremes, Salben und dergleichen. Dabei können unpolare und polare Ölkomponenten gleichermaßen eingesetzt werden. Die Ölkörper sind üblicherweise in einer Gesamtmenge von 0,1 - 50 Gew.-%, vorzugsweise 5 - 25 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen in Betracht. Daneben eigenen sich auch Ester von linearen $C_6$-$C_{22}$-Fettsäuren mit unverzweigten oder verzweigten mehrwertigen Alkoholen wie z.B. Butylene Glycol Dicaprylat/Dicaprat (Dermofeel® BGC) oder Ester des 2-Methyl-1,3-propandiols, des 1,1,1-Trimethylolethans oder 1,1,1-Trimethylolpropans. Desweiteren eignen sich Ester von linearen $C_6$-$C_{22}$-Fettsäuren mit verzweigten Alkoholen, insbesondere 2-Ethylhexanol oder Isoamylalkohol, Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis von $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen, Siliconöle (Cyclomethicone, Sliciummethicontypen u. a.) und/oder aliphatische bzw. naphthenische Kohlenwasserstoffe, wie z. B. Squalan, Squalen oder Dialkylcyclohexane.

Desweiteren können Emulsionen, welche mit Hilfe des erfindungsgemäßen Emulgatorsystems hergestellt wurden, noch weitere Zusätze wie z.B. Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektrepellentien, Selbstbräuner, Tyrosininhibitoren (Depigmentierungsmittel) , Hydrotrope, Solubilisatoren, Konservierungsmittel, Parfümöle, Farbstoffe etc. enthalten, von denen einige nachstehend exemplarisch aufgelistet sind.

Emulgatoren

Als zusätzlicher Emulgatoren kommen beispielsweise nichtionogene Tenside aus mindestens einer der folgenden Gruppen in Frage:

- Anlagerungsprodukte von 2 bis 30 Mol Ethylenoxid und/oder 0 bis 5 Mol Propylenoxid an linear Fettalkohole mit 8 bis 22 C-Atomen, an Fettsäuren mit 1 bis 22 C-Atomen, an Alkylphenole mit 8 bis 15 C-Atomen in der Alkylgruppe sowie Alkylamine mit 8 bis 22 Kohlenstoffatomen im Alkylrest

- Alkyloligoglykoside mit 8 bis 22 Kohlenstoffatomen im Alkylrest und deren ethoxylierte Analoga

- Anlagerungsprodukte von 1 bis 15 Mol Ethylenoxid an Ricinusöl und/oder gehärtetes Ricinusöl

- Anlagerungsprodukte von 15 bis 60 Mol Ethylenoxid an Ricinusöl und/oder gehärtetes Ricinusöl

- Partialester von Glycerin und/oder Sorbitan mit ungesättigten, linearen oder gesättigten, verzweigten Fettsäuren mit 12 bis 22 Kohlenstoffatomenund/oder Hydroxyarbonsäuren mit 3 bis 18 Kohlenstoffatomen sowie deren Addukte mit 1 bis 30 Mol Ethylenoxid.

- Partialester von Polyglycerin (durchschnittlicher Eigenkondenstationsgrad 2 bis 8), Polyethylenglycol (Molekulargewicht 400 bis 5000), Trimethylolpropan, Pentaerythrit, Zuckeralkoholen (z. B. Sorbit), Alkylglucosiden (z. B. Methylglucosid, Butylglucosid, Laurylglucosid) sowie Polyglucosiden (z. B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid

- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin

- Mono-, Di- und Trialkylphosphate sowie Mono-, Di- und/oder Tri-PEG-alkylphosphate und deren Salze

- Wollwachsalkohole

- Polysiloxan-Polyalkyl-Polyether-Copolymere bzw. Entsprechende Derivate

- Block-Copolymere z. B. Polyethylenglycol-30-Dipoly-hydroxystearat

- Polymeremulgatoren, z. B. Pemulen-Typen (TR-1, TR-2) von Goodrich

- Polyalkylenglycole sowie

- Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12/18}$-Fettsäuremono- und diester von Anlagerungsprodukten von Ethylenoxid an Glycerin sind als Rückfettungsmittel für kosmetische Zubereitungen bekannt.

- Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansequioleat, Sorbitandioleat, Sorbitantrioleat, Sorbitanmonoerucat, Sorbitansesquierucat, Sorbitandierucat, Sorbitantrierucat, Sorbitanmonoricinolat, Sorbitansesquiricinolat, Sorbitandiricinolat, Sorbitantriricinolat, Sorbitanmonohydroxystearat, Sorbitanmonotartrat, Sorbitansesquitartrat, Sorbitanditartrat, Sorbitantritartrat, Sorbitanmonocitrat, Sorbitansesquitartrat, Sorbitandicitrat, Sorbitantricitrat, Sorbitanmonomaleat, Sorbitansesquimaleat, Sorbitandimaleat, Sorbitantrimaleat sowie deren technische Gemische . Ebenfalls geeignet sind Anlagerungsprodukte von 1 bis 30, vorzugsweise 5 bis 10 Mol Ethylenoxid an die genannten Sorbitanester.

- Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäure mit 12 bis 22 Kohlenstoffatomen, wie beispielsweise Azelainsäure oder Sebacinsäure.

- Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-NN-dimethylammonium-glycinate, beispielsweise das Kokosalkyldimethylammonium-glycinat, N-cylaminopropyl-N,N-dimethylammoniumglycinate, beispielsweise das Kokosacyl-aminopropyldimethylammoniumglycinate, und 2-Alkyl-3-carboxymethyl-3-hydroxy-ethylimidazoline mit jeweils 8 bis 18 C-Atomen in der Alkyl- oder Acylgruppe sowie das Kokosacylaminoethylhydroxyethylcarboxymethylglycinate. Besonders geeignet ist das unter der CTFA-Bezeichnung Cocoamidopropyl Betaine bekannte Fettsäure-amid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl - oder Acylgruppe im Molekül mindestens eine freie Aminogruppe und mindestens eine -COOH oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren N-Alkyliminidipropionäuren, N-Hydroxyethyl-N-alkylamido-propylglycin, N-Alkyltaurine, N-Alkylsarcosine, 2-Alkylaminopropionsäuren und Alkylminoessigsäuren mit jeweilsetwa 8 bis 18 C-Atomen in der Alkylgruppe. Besonders bevorzugte ampholytische Tenside sind das N-Kokosalkylaminopropionat, das Kokosacylamino-ethylaminopropionat und das $C_{12/18}$-Acylsarcosin. Schließlich kommen auch Kationtenside als Emulgatoren in Betracht, wobei solche vom Typ des Esterquats, vorzugsweise methylquaternierte Difettsäuretriethanolaminester-Salze, besonders bevorzugt sind.

Fett und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d.h. feste oder flüssige, pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwchse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher häufig auch als phosphatidylcholine (PC) bezeichnet. Als Beispiele für natürliche Lecithine seien die Kephaline genannt, die auch als phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierten Carbonsäuren mit Fettalkoholen mit 6 bis 22 Kohlenstoffatomen, speziell langkettige Ester der weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 - 15 Kohlenstoffatomen und 2 bis 10 Hydroxylgruppen sowie deren Mischungen.

Konsistenzgeber und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Bettracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (Hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und diester von Fettsäuren, Polyacrylate (z. B. Carbopole® und Permulene-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z. B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit geeigneter Homologenverteilung oder Alkyloligoglucoside sowie Elektrolyte wie Kochsalz und Ammoniumchlorid.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Stabilisatoren

Als Stabilisatoren können Metallsalze von Fettsäuren, wie z. B. Magnesium-, Aluminium- und/oder Zinkstearat bzw. ricinoleat eingesetzt werden.

Polymere

Geeignete kationische Polymere sind beispielsweise kationische Cellulosederivate, wie z. B. eine quaternisierte Hydroxyethylcellulose, die unter der Bezeichnung Polymer JR 400® von Amerchol erhältlich ist, kationische Stärke, wie z.B. quaternisierte Stärken, die unter dem Markennamen Amylomer® und symbio®quat von Dr. Straetmans erhältlich sind, Copolymere von Diallylammoniumsalzen und Acrylamiden, quaternierte Vinylpyrrolidon/Vinilimidazol-Polymere, wie z. B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z. B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlösliche Polymere, kationische Chitinderivate wie beispielsweise quaterniertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, kationisches Guar-Gum, wie z. B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ AcrylatCopolymere, Octylacrylamid/Methylmethacrylat/ tert.-Butylaminoethylmeth-acrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/ Vinylcaprolactam -Terpolymere sowie gegebenenfalls derivatisierte Celluloseether und Silicone in Frage.

Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

UV-Lichtschutzfilter und Antioxidantien

Die erfindungsgemäßen Emulgatoren können auch in hervorragender weise zur Herstellung von Sonnenschutzprodukten verwendet werden. Diese enthalten UVA und UVB-Filter welche öllöslich oder wasserlöslich sein können. Als öllösliche Substanzen sind z.B. zu nennen:

- 3-Benzylidencampher bzw. 3-3-Benzylidennorcampher und dessen Derivate, z.B. 3-(4-Methylbenzyliden)campher wie in der Patentschrift EP 0693471 B1 beschrieben.
- 2,4,6-Trianilino-p-(carbo-2'-ethylhexyl-1'oxy)-1,3,5-triazin , 1-(4-tert-butylphenyl)-3-(4-methoxyphenyl)-propan-1,3-dion , 2-[-4-(Diethylamino)-2-hydroxybenzoyl] benzoesäurehexylester, Bis-Octyloxyphenolmethoxyphenyltriazin (INCI: Bis-Ethylhexyloxyphenol Methoxyphenyl Triazine),
- 4-Aminobenzoesäurederivate, vorzugsweise 4-(Dimethylamino)benzoesäure-2-ethylhexylester, 4-(Dimethylamino) benzoesäure-octylester und 4-(Dimethylamino)benzoesäure-2-amylester.
- Ester der Zimtsäure, vorzugsweise 4-Methoxyzimtsäure-2-ethylhexylester, 4-Methoxyzimtsäure-propylester, 4-Methoxyzimtsäure-isoamylester oder 2-Cyano-3,3-phenylzimtsäure-2-ethylhexylester (Octocrylene)
- Ester der Salicylsäure, vorzugsweise Salicylsäure-2-ethylhexylester, Salicylsäure-4-isopropylbenzylester oder Salicylsäurehomomenthylester
- Derivate des Bezophenons, vorzugsweise 2-Hydroxy-4-methoxybezophenon, 2-Hydroxy-4'-methoxybezophenon, 2,2'-Dihydoxy-4-methoxybenzophenon
- Ester der Benzalmalonsäure, vorzugsweise 4-Methoxy-benzmalonsäure-di-2-ethylhexylester

- Triazinderivate wie z.B. Dioctyl Butamido Triazon (Uvasorb HEB)
- Ketotricyclo(5.2.1.0)decan-Derivate, wie in der EP 0694521 B1 beschrieben.

Als wasserlösliche Substanzen kommen in Frage:

- 2-Phenylbenzimidazol-5-sulfonsäure und deren Alkali-, Erdalkali-, Ammonium-, Alkylammonium, Alkanolammonium - und Glucammoniumsalze

- Sulfonsäurederivate von Benzophenon, vorzugsweise 2-Hydroxy-4-methoxybenzophenon-5-sulfonsäure und ihre Salze

- Sulfonsäurederivate des Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-Phenyl-3-(4'isopropylphenyl)-propan-1,3-dion sowie Enaminverbindungen, wie beschrieben in der DE 19712033 A1. Die UV-A- und UV-B- Filter können selbstverständlich auch in Mischungen eingesetzt werden. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium, Alkanolammonium- und Glucammonumsalze kombiniert.

Neben den genannten löslichen Stoffen kommen für diesen Zweck auch unlösliche Lichtschutzpigmente, nämlich feindisperse Metalloxide bzw. salze in Frage. Beispiele für geeignete Metalloxide sind insbesondere Titandioxid und Zinkoxid und daneben Oxide des Eisens, Zirkoniums, Siliziums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen du dekorative Kosmetik verwendet. Die Partikel sollen dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobisiert vorliegen. Typische Beispiele sind gecoatete Titandioxide, wie z.B. Titandioxid T 805 der Fa. Degussa oder Eusolex T 2000 der Fa. Merck. Als hydrophobe Coatingmittel kommen dabei vor allem Silicone und dabei speziell Trialkoxyoctylsilane oder Simethicone in Frage. In Sonnenschutzmitteln werden bevorzugt so genannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn schädliche UV-Strahlung in die Haut eindringt. Typische Beispiele sind hierfür Aminosäure(z.B. Histidin, Tyrosin oder Tryptophan) und deren Derivate, Imidazole (z.B. Urocaninsäure) und deren Derivate, Peptide wie D,L-Carnosin, D-Carnosin, L-Carnosin und deren Derivate, Carotinoide, Carotine (z.B. $\alpha$-Carotin, $\beta$-Carotin, Lycopin) und deren Derivate, Liponsäuren und deren Derivate (z.B. Dihydroliponsäure), Aurothioglucose, Propylthiouracil und andere Thiole (z.B. Thioredoxin, Glutathion, Cystein, Cystin, Cysteamin un deren Glycolsyl-, N-Acetyl-, Mehyl-, Ethyl-, Propyl-, Amyl-, Butyl-, Lauryl-, Palmitoyl-, Oleyl-, $\gamma$-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Butioninsulfoine, Penta-, Hexa-, Heptathioninsulfoximin) in sehr geringen verträglichen Dosierungen (z.B. pmol bis mol), ferner Komplexbildener wie z.B. $\alpha$-Hydroxyfettsäuren, Palmitinsäure, Phytinsäure, Lactoferrin), $\alpha$-Hydroxysäurem (z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbyl Palmitat, Magnesium Ascorbylphosphat, Ascobylacetat), Tocopherole (z.B. $\alpha$-Tocopherol, $\beta$-Tocopherol, $\gamma$-Tocopheol $\delta$-Tocopherol) und deren Derivate (Tocopherly Acetat), Vitamin A und Derivate (Vitamin A Palmitat) soweie Koniferylbenzoat des Benzoeharzes, Rutinsäure und deren derivate, $\alpha$-Glycosylrutin, Ferulasäure, Furfurylidengluticol, Carnosin, Butylhydrxytoluol, Butylhydroxyanisol, Nordihydroguarjakharzsäure, Nordihydroguarjaretsäure, Trihydroxybutyrophenon, Harnsäure und deren Derivate, Mannose und deren Derivate, Speroxid-Dismυtase, Zink und dessen Derivate (ZnO, $ZnSO_4$), Selen un dessen Derivate (z.B. Selen-Methionin), Stilbene und deren Derivate (z.B. Stilbenoxid, trans-Stilbenoxid) und die errfindungsgemäß geeigneten Derivate (Salze, Ester, Ether, Zucker, Nukleotide, Nukleoside, Peptide und Lipide) dieser genannten Wirkstoffe.

Biogene Wirkstoffe

Unter biogenen Wirkstoffn sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Deodorantien und keimhemmende Mittel

Kosmetische Deodorantien (Desodorantien)wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

- Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutyl-carbamat, Chlorhexidin, 3,4,'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DGMC), Salicylsäure-N-alkylamide wie z. B. Salicylsäure-n-octylamid oder Salicylsäure-n-decylamid.

- Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

- Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfumöle, die zusätzlich zu ihrer Funktion als Geruchsüberdecker den Deodorantien ihre jeweilige Duftnote verleihen. Als Parfumöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropinonat, und Benzylsalicylat. Zu den Ethern zähln beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten vrwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Couer, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen eingesetzt.

- Antitranspirantien

Antitranspirantien (Antiperspipantien) reduzieren durch Beeinflussung der Aktivität der ekkrinen Schweißdrüsen die Schweißbildung und wirken somit Achselnässe und Körpergeruch entgegen. Wässrige oder wasserfreie Formulierungen von Antitranspirantien enthalten typischerweise folgende Inhaltsstoffe:

- adstringierende Wirkstoffe
- Ölkomponenten
- nichtionische Emulgatoren
- Co-emulgatoren
- Konsistenzgeber
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringen Mengen enthalten sein. Solche öllöslichen Hilfsmittel können z. B. sein:

- entzündungshemmende, hautschützende oder wohlriechende ätherische Öle
- synthetische hautschützende Wirkstoffe und/oder
- öllösliche Parfumöle

Übliche wasserlösliche Zusätze sind z. B. Konservierungsmittel, wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose- und Stärkederivate, Kollagen, Hyaluronsäure bzw. deren Salze und ähnliche Verbindungen.

Quellmittel

Als Quellmittel für wässrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R. Lochhead in Cosm. Toil. 108, 95 (1993) entnommen werden.

Insekt-Repellentien

Als Insekt-Repellentien kommen beispielsweise N,N-Diethyl-m-toluamid, 1,2-Pentandiol oder 3-(N-n-Butyl-N-acetyl-amino)-propionic aid ethyl ester), welches unter der Bezeichnung Insect Repellent® 3535 von der Merck KGaA vertrieben wird, sowie Butylacetylaminopropionate in Frage.

Selbstbräuner und Depigmentierungsmittel

Als Selbstbräuner eignet sich Dihydroxyaceton und Erythrulose. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Hydrotrope

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Typische Beispiele sind:

- Glycerin

- Alkylenglycole, wie beispielsweise Ethylenglycol, Diethylenglycol, Propylenglycol, Butylenglycol, Hexylenglycol, 1,2-Hexandiol und Caprylyl Glycol sowie Polyethylenglycole mit einem durchschnittlichen Molekulargewicht von 100 bis 1.000 Dalton.

- technische Oligoglyceringemische mit einem Eigenkondensationsgrad von 1,5 bis 10 wie etwa technische Diglyceringemische mit einem Diglyceringehalt von 40 bis 50 Gew.-%

- Methylolverbindungen, wie insbesondere Trimethylolethan, Trimethylolpropan, Trimethylolbutan, Pentaerythrit und Dipentarythrit

- Niedriglkylglucoside, insbesondere solche mit 1 bis 8 Kohlenstoffen im Alkylrest, wie bespielsweise Methyl- und Butylglucosid

- Zuckeralkohole mit 5 bis 12 Kohlenstoffatomen, wie beispiele Sorbit oder Mannit

- Zucker mit 5 bis 12 Kohlenstoffatomen, wie beispielsweise Glucose oder Saccharose

- Aminozucker, wie beispielsweise Glucamin

- Dialkoholamine, wie Diethanolamin oder 2-Amino-1,3-propandiol

Konservierungsmittel

Als Konservierungsmittel eignen sich beispielsweise Phenoxyethanol, Parabene, Benzoesäure oder Sorbinsäure sowie die in Anlage 6, Teil A und B der Kosmetikverordnung aufgeführten weiteren Stoffklassen.

Parfümöle und Aromen

Als Parfümöle ein genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange),Wurzeln (Macis, Angelica, Sellerie, Kardamom, Costus, Iris, Calmus), Hölzern (Pinien- Sandel- Guajak-, Zedern-, Rosenholz), Kräutern und Grsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclo-hexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone, α-Isomethylionon und Methylcedrylketon, zu den Alkoholen Citronellol, Eugenol, Isoeugenol, Geraniol, Linalool, Phenylethylalkohol, 3-Phenyl-1-propanol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydrmyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambraxon, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Auch Duft- und Aromastoffe aus der Gruppe der organischen Säuren, wie z.B. p-Anissäure, 4-Oxopentansäure oder Zimtsäure können zur Beduftung von Formulierungen auf Basis der erfindungsgemäßen Emulgatoren herangezogen werden. Letztere können darüber hinaus zur mikrobiologischen Stabilisierung der Formulierungen beitragen.

Farbstoffe

Als Farbstoffe können die für kosmetische Zwecke geeigneten und zugelassenen Substanzen verwendet werden. Beispiele sind Kochenillerot A (C.I. 16255), Patentblau V (C.I. 42051), Indigotin (C.I. 73015), Chlorophyllin (C.I. 75810), Chinolongelb (C.I. 47005), Titandioxid (C.I. 77891), Indanthrenblau RS (C.I. 69800) und Krapplack (C.I. 58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Das Verhältnis der Gewichtanteile der Basisemulgatoren a) und der Konsistenzgeber b) liegt vorzugsweise in einem Bereich von 1:5 bis 5:1, weiter vorzugsweise 1:2 bis 2:1.

Das oder die Benetzmittel c) sind in einer Konzentration von 2 - 15 %, bevorzugt 5 - 10 % in dem Emulgatorsystem enthalten.

Bei dem Basisemulgator a) kann es sich bevorzugt um Glyceryl Stearate Citrate, Polyglycerin-3 Stearate und/ oder Sodium Stearoyl Lactylate handeln. Als Konaistenzgeber bzw. Co-Emulgatoren b) können bevorzugt Cetylalkohol, Stearylalkohol oder Glyceryl Monostearate verwendet werden. Als Benetzmittel c) werden bevorzugt bspw. Glyceryl Caprylate oder 1,2-Octandiol (Caprylyl Glycol) verwendet. Ein bevorzugtes Emulgatorsystem enthält Glyceryl Stearate Citrate, Cetylalkohol, Stearylalkohol und Glyceryl Caprylate.

Gegenstand der Erfindung ist ferner die Verwendung eines erfindungsgemäßen Emulgatorsystems bei der Herstellung kosmetischer und/oder dermatologischer Produkte zur Hautpflege oder -reinigung sowie entsprechende Produkte.

Gegenstand der Erfindung ist ferner ein Verfahren zur Herstellung eines erfindungsgemäßen Produktes wie in Anspruch 10 beschrieben, bei dem die Emulgierung bei einer Temperatur von maximal 50°C, vorzugsweise 40°C durchgeführt wird.

Ausführungsbeispiele der Erfindung und Vergleichsbeispiele werden nachfolgend erläutert

Emulgator 1 (Vergleichsbeispiel):

    50% Glyceryl Stearate Citrate
    50% Cetearylalkohol

Emulgator 2 (Vergleichsbeispiel):

    25% Polyglyceryl-3 Stearate
    25% Sodium Stearoyl Lactylate
    50 % Glyceryl Stearate

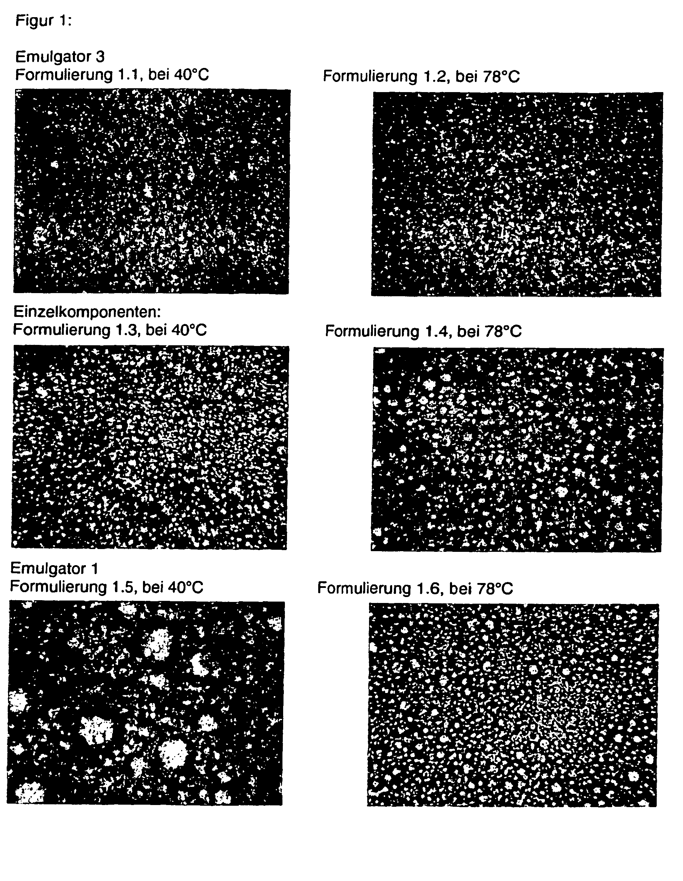

Werden diese Emulgatoren in Emulsionsprozessen bei Temperaturen zwischen 70 und 80°C eingesetzt, so bilden sich in der Regel stabile Emulsionen aus, die gegebenenfalls durch Gelbildner oder Verdicker zusätzlich stabilisiert werden können. Ein Zusatz der oben beschriebenen Benetzmittel führt bereits in niedrigen Konzentrationen zur Ausbildung sehr feiner Emulsionen mit homogener Tröpfchengrößenverteilung (siehe Figur 1)

Werden die Emulsionsprozesse jedoch bei 40°C durchgeführt, so bilden sich mit den exemplarisch angeführten Emulgatoren keine stabilen Emulsionen aus. Auch der Zusatz von Benetzmitteln zur wässrigen oder der lipophilen Phase führt zu keiner Verbesserung des Emulsionsbildungsprozesses (siehe Figur 1).

Überraschenderweise wurde nun gefunden, dass jedoch durch das zusätzliche Einschmelzen von 2 - 15 %, bevorzugt 5 - 10 % eines oder mehrere der o.g. Benetzmitteln feste Emulgatorsysteme gebildet werden, welche bereits bei Temperaturen von 40°C in der Lage sind, stabile Emulsionen mit homogener und feiner Tröpfchenverteilung auszubilden. Hierzu wurden folgende erfindungsgemäße Emulgatorsysteme hergestellt und in einer O/W-Testemulsion untersucht:

Emulgator 3 (erfindungsgemäß):

50% Glyceryl Stearate Citrate
45% Cetearylalkohol
5% Glyceryl Caprylate

Emulgator 1 und 3 wurden zur Herstellung von Hautpflegeprodukten (O/W-Emulsion) verwendet, wie in der nachfolgenden Tabelle O/W-Testemulsion 1 beschrieben. Von den Testemulsionen 1.1 bis 1.6 wurden Mikroskopaufnahmen angefertigt, die in Figur 1 dargestellt sind. Die Herstellung der Emulsionen wurde wie folgt durchgeführt:

**40°C Prozess:**

Phase A wurde auf 40°C erwärmt und Keltrol eindispergiert. Phase B1 wurde auf 40°C erwärmt. Phase B1 wird unter Rühren zu Phase A hinzugefügt gefolgt von Phase B. Die resultierende Mischung wurde für 10 Minuten kräftig gerührt und anschließend mit dem Ultra Turrax für 3-5 Minuten homogenisiert. Die Emulsion wurde anschließend unter Rühren auf Raumtemperatur abgekühlt und mit Phase C auf den angegebenen pH-Wert eingestellt

**78°C Prozess:**

Phase A wurde auf 78°C erwärmt und Keltrol eindispergiert. Die Phasen B und B1 wurden gemeinsam auf 78°C erwärmt und unter Rühren wird zur Phase A hinzugefügt. Die resultierende Mischung wurde für 1-2 Min. mit dem Ultra Turrax homogenisiert, unter Rühren bei mittlerer Geschwindigkeit auf Raumtemperatur abgekühlt und mit Phase C auf den angegebenen pH-Wert eingestellt

| O/W-Testemulsion 1 | | | 1.1 | 1.2. | 1.3 | 1.4 | 1.5 | 1.6 |
|---|---|---|---|---|---|---|---|---|
| | | Herstelltemperatur | 40°C | 78°C | 40°C | 78°C | 40°C | 78°C |
| Phase | Ingredient | INCI | % | % | % | % | % | % |
| A | Wasser | Aqua | 72,50 | 72,50 | 72,35 | 72,32 | 72,37 | 72,39 |
| | Glycerol | Glycerin | 4,00 | 4,00 | 4,00 | 4,00 | 4,00 | 4,00 |
| | bermoteel PA-3 | . Sodium Phytate | 0,10 | 0,10 | 0,10 | 0,10 | 0.10 | 0,10 |
| | Dermosoft 1388 | Glycerin, Aqua, Partum | 3,00 | 3,00 | 3,00 | 3,00 | 3,00 | 3,00 |
| A1 | Keltrol RD | Xanthan Gum | 0,20 | 0,20 | 0,20 | 0,20 | 0,20 | 0,20 |

(fortgesetzt)

| O/W-Testemulsion 1 | | | 1.1 | 1.2. | 1.3 | 1.4 | 1.5 | 1.6 |
|---|---|---|---|---|---|---|---|---|
| | | Herstelltemperatur | 40°C | 78°C | 40°C | 78°C | 40°C | 78°C |
| Phase | Ingredient | INCI | % | % | % | % | % | % |
| B | Emulgator 3 | Glyceryl Stearate Citrate, Cetearyl Alcohol, Glyceryl Caprylate | 5,00 | 5,00 | | | | |
| | Emulgator 1 | Glyceryl Stearate Citrate, Cetearyl Alcohol | | | | | 4,75 | 4,75 |
| | Dermofeel GSC | Glyceryl Stearate Citrate | | | 2,50 | 2,50 | | |
| | Dermosoft GMCY | Glyceryl Caprylate | | | 0,25 | 0,25 | 0,25 | 0,25 |
| | Lanette O | Cetearyl Alcohol | | | 2,25 | 2,25 | | |
| B1 | Tegosoft DC | Decyl Cocoate | 5,00 | 5,00 | 5,00 | 5,00 | 5,00 | 5,00 |
| | Sun Flower Seed Oil | Helianthus Annuus | 4,00 | 4,00 | 4,00 | 4,00 | 4,00 | 4,00 |
| | Dermofeel MCT | Tricaprylin | 6,00 | 6,00 | 6,00 | 6,00 | 6,00 | 6,00 |
| | | | | | | | | |
| | Dermofeel Toco 70 non-GMO | Tocopherol | 0,20 | 0,20 | 0,20 | 0,20 | 0,20 | 0,20 |
| | | | | | | | | |
| C | Citric Acid (20%ig) | Citric Acid | | | 0,15 | 0,18 | 0,13 | 0,11 |
| | | | 100% | 100% | 100% | 100% | 100% | 100% |
| | | | | | | | | |
| | | pH | 5,55 | 5,47 | 5,47 | 5,39 | 5,47 | 5,45 |
| | | Aussehen | Homogen | Homogen | Partikel | Homogen | Partikel | Homogen |
| | | Zentrifuge | i.O. | i.O. | n.i.O. | i.O. | n.i.O. | i.O. |
| | | Viskosität [mPa·s] | 4000 | 16000 | 3500 | 6000 | 5000 | 20000 |
| | | Mikroskop | Siehe Figur 1 | Siehe Figur 1 | Siehe Figur 1 | Siehe Figur 1 | Siehe Figur 1 | Siehe Figur 1 |
| | Stabilität | 50°C, 10 Tage | i.O. | i.0 | n.i.O. Wasser | i.O. | n.i.O. Wasser | i.O. |

Die eingesetzen Rohstoffe wurden aus folgende Quellen bezogen: Dermosoft® und Dermofeel® sind registrierte Marken der Dr. Straetmans GmbH, Lanette O wurde von der Firma Cognis bezogen, Tegosoft DC von der Firma Evonik Goldschmidt und Keltrol RD von der Firma CP Kelco. Die Testergebnisse in den Tabellen bedeuten folgendes:

Testergebnisse Zentrifuge und Stabilität:

i.O. bedeutet keine Auffälligkeiten
n.i.O. bedeutet Wasserabscheidungen

Auch die Verwendung von 1,2-Octandiol als Benetzmittel eignet sich in hervorragender Weise den gewünschten Effekt zu erzielen, wie die erfindungsgemäßen Beispiele mit dem Emulgatorsystem 4 demonstrieren

Emulgator 4 (erfindungsgemäß):

50% Glyceryl Stearate Citrate
42,5 % Cetyl Alkohol
7,5 % Caprylyl Glycol

| O/W-Testemulsion 2 | | | 2.1 | 2.2. | 2.3 | 2.4 |
|---|---|---|---|---|---|---|
| | | Herstelltemperatur | 40°C | 78°C | 40°C | 78°C |
| Phase | Ingredient | INCI | % | % | % | % |
| A | Wasser | Aqua | 72,50 | 72,50 | 72,35 | 72,32 |
| | Glycerol | Glycerin | 4,00 | 4,00 | 4,00 | 4,00 |
| | Dermofeel PA-3 | Sodium Phytate | 0,10 | 0,10 | 0,10 | 0,10 |
| | Dermosoft 1388 | Glycerin, Aqua, Parfum | 3,00 | 3,00 | 3,00 | 3,00 |
| A1 | Keltrol RD | Xanthan Gum | 0,20 | 0,20 | 0,20 | 0,20 |
| B | *Emulgator 4* | *Glyceryl Stearate Citrate, Cetearyl Alcohol, Caprylyl Glycol* | *5,00* | *5,00* | | |
| | *Dermofeel GSC* | *Glyceryl Stearate Citrate* | | | *2,50* | *2,50* |
| | *Dermosoft GMCY* | *Glyceryl Caprylate* | | | *0,37* | *0,37* |
| | *Lanette O* | *Cetearyl Alcohol* | | | 2,13 | 2,13 |
| B1 | Tegosoft DC | Decyl Cocoate | 5,00 | 5,00 | 5,00 | 5,00 |
| | Sun Flower Seed Oil | Helianthus Annuus | 4,00 | 4,00 | 4,00 | 4,00 |
| | Dermofeel MCT | Tricaprylin | 6,00 | 6,00 | 6,00 | 6,00 |
| | | | | | | |
| | Dermofeel Toco 70 non-GMO | Tocopherol | 0,20 | 0,20 | 0,20 | 0,20 |
| | | | | | | |
| C | Citric Acid (20%ig) | Citric Acid | | | 0,13 | 0,11 |
| | | | 100% | 100% | 100% | 100% |

(fortgesetzt)

| O/W-Testemulsion 2 | | | **2.1** | **2.2.** | **2.3** | **2.4** |
|---|---|---|---|---|---|---|
| | | Herstelltemperatur | **40°C** | **78°C** | **40°C** | **78°C** |
| **Phase** | **Ingredient** | **INCI** | **%** | **%** | **%** | **%** |
| | | | | | | |
| | | pH | 5,48 | 5,49 | 5,50 | 5,47 |
| | | Aussehen | Homogen | Homogen | Partikel | Homogen |
| | | Zentrifuge | i.O. | i.O. | n.i.O. | i.O. |
| | | Viskosität [mPa·s] | 4000 | 21000 | 3500 | 9000 |
| | Stabilität | 50°C, 10 Tage | i.O. | i.O. | n.i.O. Wasser | i.O. |

Die eingesetzen Rohstoffe wurden aus folgende Quellen bezogen: Dermosoft® und Dermofeel® sind registrierte Marken der Dr. Straetmans GmbH, Lanette O wurde von der Firma Cognis bezogen, Tegosoft DC von der Firma Evonik Goldschmidt und Keltrol RD von der Firma CP Kelco.

Der gleiche Effekt wird auch bei anderen Kombinationen aus Basisemulgator und Co-Emulgator gezeigt, wie die erfindungsgemäßen Beispiele mit dem Emulgatorsystem 5 demonstrieren.

Emulgator 5 (erfindungsgemäß):

27,5% Polyglyceryl-3 Stearate
27,5% Sodium Stearoyl Lactylate
40% Glyceryl Stearate SE
5% Glyceryl Caprylate

| O/W-Testemulsion 3 | | | **3.1** | **3.2.** | **3.3** | **3.4** |
|---|---|---|---|---|---|---|
| | | Herstelltemperatur | **40°C** | **78°C** | **40°C** | **78°C** |
| **Phase** | **Ingredient** | **INCI** | % | % | % | % |
| **A** | Wasser | Aqua | 70,35 | 70,35 | 70,10 | 70,22 |
| | Glycerol | Glycerin | 4,00 | 4,00 | 4,00 | 4,00 |
| | Dermofeel PA-3 | Sodium Phytate, Aqua | 0,10 | 0,10 | 0.10 | 0,10 |
| | Dermosoft 1388 | Glycerin, Aqua, Parfum | 3,00 | 3,00 | 3,00 | 3,00 |
| **A1** | Keltrol RD | Xanthan Gum | 0,20 | 0,20 | 0,30 | 0,30 |
| **B** | *Emulgator 5* | *Polyglyceryl-3 Stearate, Sodium Stearoyl Lactylate, Glyceryl Stearate SE, Glyceryl Caprylate* | *7,00* | *7,00* | | |
| | *Dermofeel PS* | *Polyglyceryl-3 Stearate* | | | *1,95* | *1,95* |
| | *Dermofeel SL* | *Sodium Stearoyl Lactylate* | | | *1,95* | *1,95* |
| | *Dermosoft GMCY* | *Glyceryl Caprylate* | | | *0,30* | *0,30* |
| | *Cutina GMS SE* | *Glyceryl Stearate SE* | | | *2,80* | *2,80* |
| **B1** | Tegosoft DC | Decyl Cocoate | 5,00 | 5,00 | 5,00 | 5,00 |

(fortgesetzt)

| O/W-Testemulsion 3 | | | 3.1 | 3.2. | 3.3 | 3.4 |
|---|---|---|---|---|---|---|
| | | Herstelltemperatur | 40°C | 78°C | 40°C | 78°C |
| Phase | Ingredient | INCI | % | % | % | % |
| | Sun Flower Seed Oil | Helianthus Annuus | 4,00 | 4,00 | 4,00 | 4,00 |
| | Dermofeel MCT | Tricaprylin | 6,00 | 6,00 | 6,00 | 6,00 |
| | | | | | | |
| | Dermofeel Toco 70 non-GMO | Tocopherol | 0,20 | 0,20 | 0,20 | 0,20 |
| | | | | | | |
| C | Citric Acid (20%ig) | Citric Acid | 0,15 | 0,15 | 0,30 | 0,18 |
| | | | 100% | 100% | 100% | 100% |
| | | | | | | |
| | | pH | 5,45 | 5,44 | 5,52 | 5,47 |
| | | Aussehen | Homogen | Homogen | Partikel | Homogen |
| | | Zentrifuge | i.O. | i.O. | n.i.O. | i.O. |
| | | Viskosität [mPa·s] | 4000 | 13000 | 6000 | 36000 |
| | Stabilität | 50°C, 10 Tage | i.O. | i.O. | separiert | i.O. |

Die eingesetzen Rohstoffe wurden aus folgende Quellen bezogen: Dermosoft® und Dermofeel® sind registrierte Marken der Dr. Straetmans GmbH, Cutinia GMS SE wurde von der Firma Cognis bezogen, Tegosoft DC von der Firma Evonik Goldschmidt und Keltrol RD von der Firma CP Kelco.


**Patentansprüche**

1. Festes Emulgatorsystem, **dadurch gekennzeichnet, dass** es enthält:

   a) mindestens einen Basisemulgator, ausgewählt aus der Gruppe der Ester oder Partialester von linearen oder verzweigten, gesättigten oder ungesättigten Fettsäuren mit einer Kettenlänge von n = 16 - 22 Kohlenstoffatomen, und einer Alkoholkomponente ausgewählt aus der Gruppe der Lactylate, der Polyglycerine mit einem Polymerisierungsgrad von n = 2 - 10 oder dem Zitronensäureesters des Glycerins,
   b) mindestens einen Konsistenzgeber und/oder Co-Emulgator, ausgewählt aus der Gruppe der linearen oder verzweigten, gesättigten oder ungesättigten Fettalkohole mit einer Kettenlänge von n = 16 - 22 Kohlenstoffatomen und/oder den Monoglyceriden von linearen oder verzweigten, gesättigten oder ungesättigten Fettsäuren mit einer Kettenlänge von n = 16 - 22 Kohlenstoffatomen,
   c) 2-15 % mindestens eines Benetzmittels mit einem Molekulargewicht zwischen 100 und 250 g/mol und einem HLB-Wert zwischen 6 und 12, ausgewählt aus der Gruppe der Glycerinmonoester mit Fettsäuren einer Kettenlänge von n = 6 -10 Kohlenstoffatomen oder der 1,2-Alkandiole mit einer Kettenlänge n = 5 - 10 Kohlenstoffatomen.

2. Emulgatorsystem nach Anspruch 1, **dadurch gekennzeichnet, dass** es PEG-frei ist.

3. Emulgatorsystem nach Anspruch 1 oder 2, **dadurch gekennzeichnet, dass** das Verhältnis der Gewichtanteile der Basisemulgatoren a) und der Konsistenzgeber b) in einem Bereich von 1:5 bis 5:1, vorzugsweise 1:2 bis 2:1 liegt.

4. Emulgatorsystem nach einem der Ansprüche 1 bis 3, **dadurch gekennzeichnet, dass** das oder die Benetzmittel c) in einer Konzentration von 5 - 10 % enthalten sind.

5. Emulgatorsystem nach einem der Ansprüche 1 bis 4, **dadurch gekennzeichnet, dass** der Basisemulgator a) ausgewählt ist aus der Gruppe Glyceryl Stearate Citrate, Polyglycerin-3 Stearate und/oder Sodium Stearoyl Lactylate.

**6.** Emulgatorsystem nach einem der Ansprüche 1 bis 5, **dadurch gekennzeichnet, dass** der oder die Konsistenzgeber bzw. Co-Emulgatoren b) ausgewählt ist aus der Gruppe Cetylalkohol, Stearylalkohol oder Glyceryl Monostearate.

**7.** Emulgatorsystem nach einem der Ansprüche 1 bis 6, **dadurch gekennzeichnet, dass** das Benetzmittel c) ausgewählt ist aus der Gruppe Glyceryl Caprylate und/oder 1,2-Octandiol (Caprylyl Glycol).

**8.** Emulgatorystem nach einem der Ansprüche 1 bis 7, **dadurch gekennzeichnet, dass** es Glyceryl Stearate Citrate, Cetylalkohol, Stearylalkohol und Glyceryl Caprylate enthält.

**9.** Verwendung eines Emulgatorsystems nach einem der Ansprüche 1 bis 8 bei der Herstellung kosmetischer und/oder dermatologischer Produkte zur Hautpflege oder -reinigung.

**10.** Verfahren zur Herstellung eines kosmetischen oder dermatologischen Produktes mit den Schritten:

a) zur Verfügung steller der zu emulgierenden Bestandteile des Produktes,
b) zur Verfügung stellen eines Emulgatorsystems nach einem der Ansprüche 1 bis 8,
c) Herstellen einer Emulsion unter Einsatz der Bestandteile a) und b) bei einer Temperatur von höchstens 50°C, vorzugweise höchstens 40°C.

**11.** Verfahren nach Anspruch 10, **dadurch gekennzeichnet, dass** das Emulgatorsystem in einem Anteil von 2 - 10%, vorzugsweise 3 - 8%, weiter vorzugsweise 4 - 7%, weiter vorzugsweise 5 - 6% eingesetzt wird.

**12.** Verfahren nach Anspruch 10 oder 11, **dadurch gekennzeichnet, dass** das Produkt einen Ölkörperanteil von 0,1 - 50%, vorzugsweise 5 - 25% aufweist.


## Claims

**1.** A solid emulsifier system, **characterised in that** it contains:

a) at least one base emulsifier selected from the group of esters or partial esters of linear or branched, saturated or unsaturated fatty acids with of a chain length of n = 16-22 carbon atoms, and an alcohol component selected from the group of lactylates, polyglycerols with a degree of polymerisation of n = 2-10 or the citric acid ester of glycerol,
b) at least one consistency provider and/or co-emulsifier selected from the group of linear or branched, saturated or unsaturated fatty alcohols with of a chain length of n = 16-22 carbon atoms and/or the monoglycerides of linear or branched, saturated or unsaturated fatty acids with a chain length of n = 16-22 carbon atoms,
c) 2-15% of at least one wetting agent with a molecular weight between 100 and 250 g/mol and an HLB value between 6 and 12, selected from the group of glycerol monoesters with fatty acids of a chain length of n = 6-10 carbon atoms or of 1,2-alkanediols with a chain length of n = 5-10 carbon atoms.

**2.** An emulsifier system according to claim 1, **characterised in that** it contains no PEG.

**3.** An emulsifier system according to claim 1 or claim 2, **characterised in that** the ratio of the proportion by weight of the base emulsifier a) and of the consistency provider b) is in a range from 1:5 to 5:1, preferably 1:2 to 2:1.

**4.** An emulsifier system according to any one of claims 1 to 3, **characterised in that** the wetting agent(s) c) is/are present in a concentration of 5-10%.

**5.** An emulsifier system according to any one of claims 1 to 4, **characterised in that** the base emulsifier a) is selected from the group glyceryl stearate citrate, polyglyceryl-3 stearate and/or sodium stearoyl lactylate.

**6.** An emulsifier system according to any one of claims 1 to 5, **characterised in that** the consistency provider(s) or co-emulsifier (s) b) is/are selected from the group cetyl alcohol, stearyl alcohol or glyceryl monostearate.

**7.** An emulsifier system according to any one of claims 1 to 6, **characterised in that** the wetting agent c) is selected from the group glyceryl caprylate and/or 1,2-octanediol (caprylyl glycol).

**8.** An emulsifier system according to any one of claims 1 to 7, **characterised in that** it contains glyceryl stearate citrate, cetyl alcohol, stearyl alcohol and glyceryl caprylate.

**9.** Use of an emulsifier system according to any one of claims 1 to 8 in the production of cosmetic and/or dermatological products for skin conditioning or cleaning.

**10.** A method for producing a cosmetic or dermatological product with the steps:

a) providing the product constituents to be emulsified,
b) providing an emulsifier system according to any one of claims 1 to 8,
c) producing an emulsion using constituents a) and b) at a temperature of at most 50°C, preferably at most 40°C.

**11.** A method according to claim 10, **characterised in that** the emulsifier system is used in a proportion of 2-10%, preferably 3-8%, more preferably 4-7%, more preferably 5-6%.

**12.** A method according to claim 10 or claim 11, **characterised in that** the product has an oil body content of 0.1-50%, preferably of 5-25%.

**Revendications**

**1.** Système émulsifiant solide, **caractérisé en ce qu'**il contient :

a) au moins un émulsifiant de base, choisi dans le groupe des esters ou des esters partiels d'acides gras saturés ou insaturés à chaîne droite ou ramifiée, ayant une longueur de chaîne n = 16-22 atomes de carbone, et d'un composant alcool choisi dans le groupe des lactylates, des polyglycérols ayant un degré de polymérisation n = 2-10, ou l'ester de l'acide citrique du glycérol,
b) au moins un agent de consistance et/ou un co-émulsifiant, choisi dans le groupe des alcools gras saturés ou insaturés à chaîne droite ou ramifiée, ayant une longueur de chaîne n = 16-22 atomes de carbone, et/ou les monoglycérides d'acides gras saturés ou insaturés à chaîne droite ou ramifiée, ayant une longueur de chaîne n = 16-22 atomes de carbone,
c) 2 à 15 % d'au moins un mouillant ayant une masse moléculaire comprise entre 100 et 250 g/mol et une valeur HLB comprise entre 6 et 12, choisi dans le groupe des monoesters du glycérol d'acides gras ayant une longueur de chaîne n = 6-10 atomes de carbone, ou des 1,2-alcanediols ayant une longueur de chaîne n = 5-10 atomes de carbone.

**2.** Système émulsifiant selon la revendication 1, **caractérisé en ce qu'**il est exempt de PEG.

**3.** Système émulsifiant selon la revendication 1 ou 2, **caractérisé en ce que** le rapport des parties en poids des émulsions de base a) aux parties en poids de l'agent de consistance b) est compris dans une plage de 1:5 à 5:1, de préférence de 1:2 à 2:1.

**4.** Système émulsifiant selon l'une des revendications 1 à 3, **caractérisé en ce que** le ou les ligands c) y sont présents en une concentration de 5 à 10 %.

**5.** Système émulsifiant selon l'une des revendications 1 à 4, **caractérisé en ce que** l'émulsifiant de base a) est choisi dans le groupe du stéarate et citrate de glycéryle, du stéarate de polyglycérol-3 et/ou du stéaroyllactylate de sodium.

**6.** Système émulsifiant selon l'une des revendications 1 à 5, **caractérisé en ce que** le ou les agents de consistance ou le ou les co-émulsifiants b) sont choisis dans le groupe de l'alcool cétylique, de l'alcool stéarylique ou du monostéarate de glycéryle.

**7.** Système émulsifiant selon l'une des revendications 1 à 6, **caractérisé en ce que** le mouillant c) est choisi dans le groupe du caprylate de glycéryle et/ou du 1,2-octanediol (caprylylglycol).

**8.** Système émulsifiant selon l'une des revendications 1 à 7, **caractérisé en ce qu'**il contient du stéarate et citrate de glycéryle, de l'alcool cétylique, de l'alcool stéarylique et du caprylate de glycéryle.

**9.** Utilisation d'un système émulsifiant selon l'une des revendications 1 à 8 pour la fabrication de produits cosmétiques et/ou dermatologiques destinés aux soins ou au nettoyage de la peau.

**10.** Procédé de fabrication d'un produit cosmétique ou dermatologique, comportant les étapes suivantes :

a) mise à disposition des constituants à émulsifier du produit,
b) mise à disposition d'un système émulsifiant selon l'une des revendications 1 à 8,
c) fabrication d'une émulsion par utilisation des constituants a) et b) à une température d'au plus 50°C, de préférence d'au plus 40°C.

**11.** Procédé selon la revendication 10, **caractérisé en ce que** le système émulsifiant est utilisé selon une proportion de 2 à 10 %, de préférence de 3 à 8 %, d'une manière encore plus préférée de 4 à 7 % et d'une manière encore plus préférée de 5 à 6 %.

**12.** Procédé selon la revendication 10 ou 11, **caractérisé en ce que** le produit a une teneur en émollients de 0,1 à 50 %, de préférence de 5 à 25 %.

Figur 1:

Emulgator 3
Formulierung 1.1, bei 40°C

Formulierung 1.2, bei 78°C

Einzelkomponenten:
Formulierung 1.3, bei 40°C

Formulierung 1.4, bei 78°C

Emulgator 1
Formulierung 1.5, bei 40°C

Formulierung 1.6, bei 78°C

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0553241 B2
- EP 1790327 A1
- EP 1641904 B1
- EP 1250961 A1
- DE 102005051864 A1
- EP 2005937 A1
- WO 2007013634 A1
- EP 1911436 A2
- EP 0693471 B1
- EP 0694521 B1
- DE 19712033 A1

**In der Beschreibung aufgeführte Nicht-Patentliteratur**

- **GRIFFIN, W. C.** Classification of surface active agents by HLB. *J. Soc. Cosmet. Chem.,* 1949, vol. 1
- **R. LOCHHEAD.** *Cosm. Toil.,* 1993, vol. 108, 95